Europäisches Patentamt

European Patent Office

Office européen des brevets

(19)

(11) **EP 0 743 070 A2**

(12) **EUROPÄISCHE PATENTANMELDUNG**

(43) Veröffentlichungstag:
20.11.1996 Patentblatt 1996/47

(51) Int Cl.⁶: **A61K 38/48, A61K 38/49**

(21) Anmeldenummer: 96107917.5

(22) Anmeldetag: 17.05.1996

(84) Benannte Vertragsstaaten:
**AT BE CH DE DK ES FR GB IE IT LI NL SE**

(30) Priorität: **17.05.1995 CZ 127295**

(71) Anmelder:
• **Trnka Mudr, Frantisek**
**370 01 Ceske Budejovice (CS)**
• **Rybak, Miroslav, Dipl.-Ing.**
**149 00 Prag 11 (CS)**
• **Marek, Roman, Dipl.-Ing.**
**140 00 Prag 4 (CS)**
• **Vavra, Ladislav, Dipl.-Ing.**
**370 04 Ceske Budejovice (CS)**

(72) Erfinder:
• **Trnka Mudr, Frantisek**
**370 01 Ceske Budejovice (CS)**
• **Rybak, Miroslav, Dipl.-Ing.**
**149 00 Prag 11 (CS)**
• **Marek, Roman, Dipl.-Ing.**
**140 00 Prag 4 (CS)**
• **Vavra, Ladislav, Dipl.-Ing.**
**370 04 Ceske Budejovice (CS)**

(74) Vertreter: **Beetz & Partner Patentanwälte**
**Steinsdorfstrasse 10**
**80538 München (DE)**

(54) **Verwendung von Protease-Proenzymen und Amylasen als Wirkstoffe in pharmazeutischen Mitteln zur Krebstherapie sowie ihre Herstellung**

(57) Die Erfindung betrifft neuartige pharmazeutische Mittel mit Modulationseffekt auf maligne Geschwülste, die als Wirkstoff ein oder mehrere Protease-Proenzyme sowie vor-zugsweise noch eine oder mehrere Amylasen enthalten, günstig im Verhältnis 1:100 bis 100:1, bezogen auf die Einheiten der enzymatischen Aktivität. Die pharmazeutischen Mittel enthalten ferner vorteilhaft einen Proteaseinhibitor, wie Aprotinin. Die pharmazeutischen Mittel eignen sich zur Behandlung maligner Geschwülste in der Human- und Veterinärmedizin. Die pharmazeutischen Mittel modu-lieren die biologische Antwort des Organismus auf maligne Geschwülste. Sie können rektal, intramuskulär oder subkutan appliziert werden.

EP 0 743 070 A2

## Beschreibung

Die Erfindung betrifft pharmazeutische Mittel zur Krebstherapie, die als Wirkstoffe Protease-Proenzyme und gegebenenfalls Amylasen enthalten und zur pharmakologischen Beeinflussung der biologischen Antwort des Organismus bei der Wechselwirkung mit malignen Geschwülsten geeignet sind. Diese pharmazeutischen Mittel sind in der Humanmedizin sowie in der Veterinärmedizin anwendbar. Die Erfindung betrifft ferner die Herstellung entsprechender pharmazeutischer Mittel und nichttherapeutische Verfahren zur Behandlung maligner Geschwülste oder maligner Zellen bzw. Zellkulturen.

Bei der gegenwärtigen onkologischen Therapie werden vor allem Eradikationsmethoden vom chirurgischen Typ sowie die Strahlentherapie zur Beseitigung oder wenigstens zur Reduzierung von Primärgeschwülsten angewandt. Dabei wird vorausgesetzt, daß das natürliche Immunsystem des Organismus die restlichen, latenten Geschwulstpopulationen allein zu bewältigen vermag. Diese therapeutische Grundverfahrensweise wird durch eine adjuvante und in jüngster Zeit auch durch eine nichtadjuvante Form der Chemotherapie modifiziert. Die angewandte therapeutische Kombination ist stets ein Kompromiß zwischen dem erwünschten und dem unerwünschten immunsuppressiven Effekt aller angeführten Methoden und dem selbständigen kanzerogenen Effekt der Strahlentherapie wie auch der Chemotherapie. Daneben ist noch die Hormontherapie zu nennen, die jedoch methodisch unter die Chemotherapie zu rechnen ist und bei der ebenfalls unerwünschte, d.h., immunsuppressive, kanzerogene Wirkungen auftreten können. Der günstige Endeffekt der angeführten kombinierten Behandlungen setzt daher eine ausgeprägte Immuntoleranz des Organismus gegenüber der ablaufenden Erkrankung sowie gegenüber der Behandlung voraus. Eine solche Situation ist jedoch nicht die Regel und tritt auch nicht bei einem großen Prozentsatz der Fälle auf. Die gleichzeitig mit derartigen Behandlungen oder anschließend vorgenommenen therapeutischen Bemühungen, eine Immunmodulation bei der Immuntherapie zu erzielen, versagten grundsätzlich mit fortgeschrittener Erkrankung und aufgrund des Gewebekatabolismus. In der onkologischen Therapie ist derzeit eine Art Renaissance der Enzymtherapie festzustellen, die mit ihren historischen Wurzeln in die erste Dekade dieses Jahrhunderts zurückreicht (vgl. Beard, Chadto und Windus, London 1911). Auf diese Autoren gehen die Arbeiten von Freund und Kaminer (vgl. E. Freund, G. Kaminer, Springer-Verlag, Wien 1925; E. Freund, Med. Wschr. (1934) 12) und von Christiani (A. Christiani, Krebsforsch. 47, (1938) 176) zurück. An die theoretischen und experimentellen Erkenntnisse der angeführten Autoren knüpften später Wolf und Ransberger (vgl. M. Wolf, K. Ransberger, Enzymtherapie, Maudrich, Wien, 1970) mit klinischen Studien an. Diese Autoren bewiesen die selektive onkolytische Wirksamkeit tierischer und pflanzlicher Hydrolasen (Proteasen) und die Teilnahme von Enzymen verschiedener Provenienz bei der Potenzierung des onkolytischen Effekts. Diese Autoren stellten auch die enterale Resorption von Hydrolasen und Glycosidasen (Amylasen) fest und legten so die Grundlage für eine perorale Therapie (vgl. z.B. die Wobe-Mugos®-Präparate)

Die systemische Enzymtherapie beruht auf der klinischen Ausnutzung experimentell gewonnener Daten über die Wirksamkeit aktiver Proteasen. Es handelt sich dabei um eine entzündungshemmende systemische Wirkung und eine Beeinflussung der Fibrinolyse, die Stimulation der Cytokinbildung (TNF-$\alpha$, I1 2,6) sowie die Stimulation der Aktivität polymorphkerniger Leukozyten sowie des Makrophagensystems.

Zu den lokalen geschwulsthemmenden Wirkungen gehört der selektive onkolytische Einfluß im Sinne einer Verringerung adhäsiver Moleküle an Geschwulstzellmembranen sowie, zusammen mit der fibrinolytischen systemischen Wirksamkeit, die Verhinderung der Metastasenbildung.

Ferner sind noch die Arbeiten von Yoneda et al. zu erwähnen (vgl. T. Yoneda et al., Cancer Res. 56 (1994) 2509), die durch Verabreichung eines teilweise gereinigten Extrakts von Schweinepankreas an Mäuse mit einer menschlichen spinozellulären Hautgeschwulst eine positive Wirkung auf die Anorexie, den Gewichtsverlust, die Entwicklung der Kachexie und die Überlebensdauer nachgewiesen haben. Die Autoren verwendeten bei den Untersuchungen einen teilweise gereinigten Pankreasextrakt, ohne jedoch die Natur des Wirkstoffs genauer zu definieren.

Bei der Anwendung aktiver Proteasen zur Therapie müssen allerdings einige Umstände beachtet werden, aufgrund deren die Wirksamkeit dieser Therapie Begrenzungen unterliegt und die im Rahmen der Erfindung in Betracht gezogen wurden:

a) Pankreasproteasen werden im Blutkreislauf sowie im Interstitialraum rasch durch anwesende polyvalente Inhibitoren inaktiviert ($\alpha$-1-Inhibitor, Antithrombin III, C1-Inhibitor, Chymotrypsin-Inhibitor). Die Möglichkeit der Dissoziation des Komplexes $\alpha$-2-Makroglobulin-Protease in Gegenwart eines Substrats mit größerer Affinität für die Protease wurde für viele proteolytische Systeme (z.B. für $\alpha$-2-Makroglobulin-Plasmin-Fibrin) nachgewiesen, jedoch ist das Verständnis des Systems a-2-Makroglobulin-Trypsin-Rezeptor für Trypsin an Zellwandungen von Geschwulstzellen bisher noch in einem theoretischen bzw. hypothetischen Stadium. Als Vorteil der Bindung zwischen Inhibitor und Protease ist die Herabsetzung der Antigenwirksamkeit körperfremder Proteasen infolge der Komplexbildung anzusehen.

b) Trypsinartige Proteasen können, ebenso wie weitere Proteasen (z.B. lysosomales Kathepsin B) die Invasivität von Geschwulstzellen durch Zerstörung des angrenzenden Gewebes des Wirtsorganismus erhöhen (vgl. E. Koivanen, Int. J. Cancer 47 (4) (1991) 592).

c) Trypsin kann in das System der Blutproteasen, die miteinander durch Aktivierungs- und Inhibitionsmechanismen verknüpft sind, eingreifen (Trypsin greift in das Koagulationssystem bzw. das fibrinolytische System in unerwünschter Weise ein, setzt Kinine, die Mediatoren für Entzündungen und Schmerzen darstellen, aus plasmatischen Kininogenen frei, etc.).

Der Erfindung liegt die Aufgabe zugrunde, pharmazeutische Mittel zur Krebstherapie und insbesondere zur Behandlung maligner Geschwülste sowie deren Herstellung und Verwendung anzugeben, bei denen die oben angeführten Nachteile der herkömmlichen Enzymtherapie vermieden sind und die vorzugsweise eine gegenüber dem Stand der Technik selektivere Wirksamkeit gegenüber malignen Geschwüren bzw. entsprechenden Zellen und Zellkulturen aufweisen.

Die Aufgabe wird gemäß den unabhängigen Ansprüchen gelöst. Die abhängigen Ansprüche betreffen vorteilhafte Ausführungsformen der Erfindungskonzeption.

Die erfindungsgemäßen pharmazeutischen Mittel, die insbesondere einen Modulationseffekt auf maligne Geschwülste ausüben, sind dadurch gekennzeichnet, daß sie als Wirkstoff ein oder mehrere Protease-Proenzyme enthalten.

Nach einer besonders vorteilhaften Ausführungsform der Erfindungskonzeption enthalten die pharmazeutischen Mittel neben den Protease-Proenzymen eine oder mehrere Amylasen, insbesondere $\alpha$-Amylasen, die mit den Zuckerkomponenten der Membranen von Zellen maligner Geschwülste reagieren können.

Nach einer vorteilhaften Ausführungsform liegen das oder die Protease-Proenzyme und die Amylase(n) in einem solchen Mengenverhältnis vor, daß das Verhältnis der enzymatischen Aktivität der Protease-Proenzyme zur enzymatischen Aktivität der Amylase(n), jeweils ausgedrückt in entsprechenden Einheiten der enzymatischen Aktivität, im Bereich von 1:100 bis 100:1 liegt.

Es ist erfindungsgemäß bevorzugt, als Protease-Proenzyme ein oder mehrere Proenzyme einzusetzen, die unter Trypsinogen, Chymotrypsinogen, Proelastase und Prokallikrein ausgewählt sind.

Es sind jedoch auch beliebige andere Proenzyme (Zymogene) anwendbar, beispielsweise Pepsinogen, Pro-Carboxypeptidazen, Plasminogen, Proreumin, Prothrombin, Pro-Urokinase, etc. Derartige Protease-Proenzyme sind als solche dem Fachmann geläufig.

Die Erfindungskonzeption beruht allgemein darauf, daß eine oder mehrere Proteasen in einer nichtaktiven Form eingesetzt werden, wodurch die oben erläuterten Nachteile der Verwendung aktiver Proteasen vermieden werden.

Eine besonders bevorzugte inaktive Form von Proteasen sind die entsprechenden Proenzyme.

Die eingesetzten Proteasen können menschlichen oder tierischen Ursprungs sein. Die verwendbaren Amylasen können ferner menschlichen, tierischen, pflanzlichen oder bakteriellen Ursprungs sein.

Nach einer weiteren bevorzugten Ausführungsform der Erfindung enthalten die pharmazeutischen Mittel als weitere Komponente einen Proteaseinhibitor und vorzugsweise einen polyvalenten Proteaseinhibitor. Bevorzugt wird hierfür Aprotinin verwendet. Hierdurch wird die Wirksamkeit der erfindungsgemäßen pharmazeutischen Mittel noch weiter erhöht, wahrscheinlich durch pharmakokinetische Zurückdrängung der Umwandlung des Proenzyms in das entsprechende Enzym.

Die erfindungsgemäßen pharmazeutischen Mittel enthalten grundsätzlich mindestens ein Protease-Proenzym als Wirkstoff, bevorzugt in Kombination mit einer oder mehreren Amylasen. Daneben können die erfindungsgemäßen pharmazeutischen Mittel auch pharmazeutisch übliche Hilfs- und/oder Trägerstoffe sowie gegebenenfalls auch weitere, von Protease-Proenzymen und Amylasen verschiedene Wirkstoffe enthalten.

Die erfindungsgemäßen pharmazeutischen Mittel liegen bevorzugt in Form von Infusionslösungen oder von Injektionslösungen vor, insbesondere zur subkutanen Injektion.

Die erfindungsgemäßen pharmazeutischen Mittel können neben der subkutanen Injektion auch durch intramuskuläre Injektion verabreicht werden. Ferner ist eine rektale Verabreichung möglich, was einen besonderen Anwendungsvorteil darstellt, da auf diese Weise eine leichte Selbstmedikation von Patienten möglich ist.

Eine bevorzugte Ausführungsform der erfindungsgemäßen pharmazeutischen Mittel ist die galenische Form von Kits-of-Parts, die mindestens aus zwei Packungseinheiten bestehen, wobei eine Packungseinheit das oder die Protease-Proenzyme und die andere Packungseinheit eine oder mehrere Amylasen, jeweils gegebenenfalls mit üblichen Hilfs- und/oder Trägerstoffen, enthalten.

Eine andere Ausführungsform entsprechender pharmazeutischer Kits-of-Parts ist aus mindestens zwei Packungseinheiten aufgebaut, wobei die eine Packungseinheit ein oder mehrere Protease-Proenzyme und gegebenenfalls eine oder mehrere Amylasen enthält, während die andere Packungseinheit ein entsprechendes Lösungsmittel bzw. eine entsprechende Lösung enthält, mit der aus den Wirkstoffen oder anderen Packungseinheit, die vorzugsweise in trockener, lyophilisierter Form vorliegen, durch Rekonstituieren eine Infusions- bzw. Injektionslösung hergestellt werden kann.

Das erfindungsgemäße Verfahren zur Herstellung der pharmazeutischen Mittel besteht prinzipiell darin, daß der bzw. die Wirkstoffe in an sich bekannter Weise, gegebenenfalls zusammen mit üblichen Hilfs- und/oder Trägerstoffen, zu galenischen Formulierungen verarbeitet werden, insbesondere zu Infusionslösungen und Injektionslösungen.

Die entsprechenden Hilfs- und Trägerstoffe sind dem Fachmann auf dem Gebiet der Galenik geläufig. Gleiches gilt für die entsprechenden Formulierungsverfahren und hierbei verwendbare Vorrichtungen.

Die Erfindungskonzeption umfaßt ferner auch die Verwendung von Protease-Proenzymen und insbesondere von Trypsinogen, Chymotrypsinogen, Proelastase und/oder Prokallikrein, gegebenenfalls in Kombination mit einer oder mehreren Amylasen, zur Herstellung pharmazeutischer Mittel zur Krebstherapie und besonders zur Behandlung maligner Geschwülste.

Die Erfindungskonzeption umfaßt ferner auch ein Verfahren zur Krebstherapie und insbesondere zur Behandlung maligner Geschwülste oder maligner Zellen bzw. Zellkulturen, das dadurch gekennzeichnet ist, daß ein oder mehrere Protease-Proenzyme, gegebenenfalls in Kombination mit einer oder mehreren Amylasen, auf die malignen Geschwülste, Zellen bzw. Zellkulturen angewandt werden, wobei zu therapeutischen Zwecken durchgeführte Verfahren zur Behandlung des menschlichen oder tierischen Körpers ausgenommen sind.

Derartige Verfahren genießen insbesondere in der Krebs-forschung Bedeutung und umfassen beispielsweise Verfahren, bei denen maligne Zellen bzw. entsprechende Zellkulturen mit den genannten Wirkstoffen behandelt werden. Dieses Verfahren umfaßt ferner auch die Behandlung maligner Geschwülste von Versuchstieren zu Forschungszwekken, die im Anschluß an die Versuche zu Untersuchungs- und Auswertungszwecken getötet werden, bei denen also der dem Patentgesetz zugrundeliegende therapeutische Zweck der Lebenserhaltung bzw. Lebensverlängerung nicht vorliegt. Die erfindungsgemäßen Verfahren, bei denen Zellen bzw. Zellkulturen behandelt werden, genießen besonderes Interesse, da sie der Vermeidung von Tierversuchen dienen.

Ein theoretischer Ausgangspunkt der vorliegenden Erfindung im Hinblick auf eine Beeinflussung von Geschwulstzellen beruht auf dem rein biologischen Zugang zur Kanzerogenese und dem Bestreben nach einer entsprechenden Beeinflussung von Geschwülsten. Die maligne Geschwulst wird nach einer der vorliegenden theoretischen Konzeptionen im Sinne einer ektopischen Schwangerschaft aufgefaßt, die aus einem gewissermaßen steckengebliebenen primordialen Genozyten im Verlauf der embryonalen Migration in eine bestimmte Gonadenanlage entstanden ist. Diese an unrichtiger Stelle vorliegende "ektopische Schwangerschaft" im Fall einer malignen Geschwulst, die ebenso auch beim männlichen Geschlecht vorliegen kann, ist mit dem Verlust von Regulationsmechanismen verbunden, die auch für den physiologischen Ablauf einer Schwangerschaft typisch sind. Es geht hier vor allem um den Regulationseinfluß des Embryos auf den Trophoblasten, der als Analogon maligner Tumore aufgefaßt wird. In einer in der Natur physiologisch einzigartigen Weise invadiert der Trophoblast, metastasiert und wächst schließlich, wenn der Embryo geschädigt ist oder gänzlich fehlt, eigenständig weiter (Molarsyndrome, komplizierte Molen bzw. Chorionkarzinome, invasive Formen von Blasenmolen). Es wurde festgestellt, daß nach Beendigung der Organogenese in der sogenannten kritischen Periode der Embryo die Proliferation des Trophoblasten beeinflußt und einstellt. Es kommt zu einer Differenzierung des Trophoblasten, zur Entstehung des Synzytiotrophoblasten sowie zum Verschwinden der zuvor starken Mitose. Wenn der Embryo fehlt oder fehlgebildet ist, kommt es nicht zu einer Differenzierung und zu einer entsprechenden Umstrukturierung des Trophoblasten. Es entsteht somit das oben erwähnte Chorionkarzinom, das als theoretisches Modell für die Entstehung maligner Tumoren angesehen wird.

In dem Bestreben, die embryolytische Regulation zu beeinflussen und nachzuahmen, wurden bereits Embryonalextrakte verwendet, von denen Pankreasextrakte von neugeborenen Lämmern, Kälbern und Ferkeln am wirkungsvollsten waren.

Später wurden auch Gemische von zwei Enzymen, Trypsin und Amylase, angewandt. Damit wurden zwar bestimmte Ergebnisse bei der Beeinflussung von Trophoblasten erzielt, jedoch war diese Methode nicht reproduzierbar (vgl. Beard, Chadto und Windus, London 1911).

Die Konzeption der vorliegenden Erfindung, also die Verwendung von Protease-Proenzymen, insbesondere von Trypsinogen, gegebenenfalls zusammen mit einer Amylase, zeichnet sich gegenüber der früher angewandten Therapie mit aktiven Enzymen durch überraschende und zugleich außerordentlich wichtige Vorteile aus:

1. Die Verwendung von aktiven Proteasen, insbesondere in der Krebstherapie, ist als nichtphysiologische Belastung des Organismus anzusehen.

2. Die Anwendung von Protease-Proenzymen als Quelle für in situ freigesetzte aktive Proteasen ist demgegenüber physiologischer als die Direktanwendung aktiver Proteasen.

3. Der Organismus ist durch die Anwendung von Protease-Proenzymen in wesentlich geringerem Maße der Initiierung unerwünschter Vorgänge ausgesetzt, beispielsweise einem Eingriff in das Kallikrein-Kinin-Kininase-System oder die Aktivierung des Koagulationssystems und des fibrinolytischen Systems, der zu einer disseminierten intravaskulären Koagulation führt, da die Möglichkeit der Einwirkung aktiver Proteasen auf Gewebe (z.B. Dickdarmgewebe) bei Anwendung von Protease-Proenzymen signifikant verringert ist.

4. Die Wechselwirkung mit Inhibitoren, die oft mit einer irreversiblen Inaktivierung von Proteasen verbunden ist, und die Entstehung zirkulierender Komplexe von Enzymen mit Inhibitoren ist im Fall der Anwendung von Protease-Proenzymen nur minimal.

Aufgrund zahlreicher Anzeichen konnte ein Modell für die Wirkungsweise der erfindungsgemäßen pharmazeutischen Mittel aufgestellt werden, das darauf beruht, daß die Aktivierung der Proenzyme unter Bildung aktiver Enzyme auf der Oberfläche von Geschwulstzellen durch Aktivatoren zustandekommt, die bei malignen Zellen anwesend sind, jedoch bei gesunden Zellen fehlen. Dieser theoretische Wirkungsmechanismus wird durch den Befund gestützt, daß in den Zellen benigner Geschwülste Proteaseinhibitoren vorliegen, nicht aber in malignen Zellen (vgl. H. Bohe, M. Bohe, M. Lindström und K. Chisson, J. Clin. Pathol. 43 (1990) 901). Bei hoch unspezifischen Proteasen, wie beispielsweise Trypsin, besteht eine große Wahrscheinlichkeit für die Spaltung von Proteinketten in der Zellwand in der Nachbarschaft basischer Aminosäuren, wie Arginin, Lysin und Histidin.

Durch das Vorliegen einer Amylase wird andererseits die Wirkung der Proteasen auf maligne Zellen in signifikanter Weise dadurch unterstützt, daß die Polysaccharidkomponenten der an der Zelloberfläche vorliegenden Glycoproteine maligner Zellen hierdurch gespalten werden.

Die Aktivierung und die Wirkung der Proteasen erfolgen auf der Oberfläche und in enger Nachbarschaft zur Geschwulstzelle. Aufgrund der Nichtreaktivität der Protease-Proenzyme führen diese entsprechend nicht zu einer Erhöhung der Invasivität der Geschwulstzellen, wie dies andererseits bei aktiven Proteasen der Fall ist.

Einige erfindungsgemäße Versuche wurden mit hochgereinigten Proenzymen (z.B. Trypsinogen, zusammen mit Amylase) durchgeführt. Gegenüber der Verwendung von Pankreasextrakten, wie sie, wie oben erwähnt, von Yoneda et al. beschrieben wurde, liegt erfindungsgemäß eine signifikant veränderte und verbesserte geschwulstmodifizierende Wirksamkeit vor. Durch die Anwendung von Proenzymen anstelle von aktiven Proteasen treten in vivo Wirkungen auf, wie man sie nur in vitro bei Einsatz von Proteasen feststellt, nämlich Onkolyse, Herabsetzung der Anzahl adhäsiver Moleküle, Erhöhung der Immunogenität der Geschwulst, Unterdrückung der Metastasenbildung). Die Wirkung der erfindungsgemäß verwendeten Protease-Proenzyme, besonders in Kombination mit einer Amylase, ist dabei besonders hervorragend. Auf diese Weise wird dem Organismus die Möglichkeit geboten, durch das eigene Immunsystem bei der Beseitigung von Geschwülsten mitzuwirken. So ist z.B. eine Behandlung mit erfindungsgemäßen pharmazeutischen Mitteln dann besonders erfolgreich, wenn keine starke zytostatische und immunsuppressive Behandlung vorausging.

Die Erfindung wird im folgenden anhand von Ausführungsbeispielen näher erläutert, deren Angaben nicht einschränkend sind.

In den Beispielen wurden, falls nichts anderes angegeben ist, die nachstehend erläuterten Methoden angewandt und die unten beschriebenen Materialien eingesetzt.

Versuchstiere

Als Versuchstiere wurden weibliche Mäuse Cs >B1$_6$ verwendet, die ein mittleres Körpergewicht von etwa 24 g aufwiesen. Die Tiere wurden in Kunststoffkäfigen gehalten und wurden mit der Diät DOS 2b gefüttert und erhielten Wasser ad libitum.

Protease-Proenzyme und Amylase:

Als Protease-Proenzyme wurden Trypsinogen aus Rinderpankreas sowie Trypsinogen aus Schweinepankreas verwendet. Das Trypsinogen (EC 3.4.21.4) aus Rinderpankreas war ein Handelsprodukt (Sigma Co, USA) und stellte ein hochgereinigtes Präparat dar (15000 BAEE-Einheiten/mg Protein nach Aktivierung zu Trypsin und 430 BAEE-Einheiten/mg Protein an aktivem Trypsin). Die BAEE-Einheiten beziehen sich auf Benzoylargininethylester (BAEE) als Substrat.

Proteingehalt: 97 %.

Das Trypsinogen aus Schweinepankreas war ein teilweise gereinigtes Präparat, das durch saure Extraktion und Aussalzen mit Ammoniumsulfat hergestellt worden war (vgl. Mansfeld et al., Organopräparate, 1958).

Als Amylase wurde eine α-Amylase (EC 3.2.1.1) aus Schweinepankreas herangezogen. Hierfür wurde ein Handelspräparat (Sigma Co, USA) verwendet, das ein hochgereinigtes Enzym darstellte, das zweimal kristallisiert war. Das Präparat enthielt 790 Einheiten/mg Protein (bestimmt durch E$_{280}$ in 1%iger Lösung).

Die Pankreasenzyme wurden durch Extraktion von frischem Schweinepankreas und Reinigung an Carboxymethylcellulose hergestellt. Die Hauptanteile bestanden aus Trypsinogen und α-Amylase. Das Verhältnis dieser beiden Enzyme wurde zur Anwendung in den Tierversuchen durch Zugabe von gereinigtem Trypsinogen oder von Amylase jeweils entsprechend eingestellt.

Alle zur Anwendung an Mäusen bestimmten Präparate lagen in Lösung vor bzw. wurden mit einer physiologischen Kochsalzlösung verdünnt.

Definition der Aktivität der Proteasen und ihrer Proenzyme und Methoden zu ihrer Bestimmung:

Das Rindertrypsinogen (Sigma Co, USA) war vom Hersteller hinsichtlich der Aktivität durch Angabe der BAEE-Einheiten spezifiziert (1 BAEE-Einheit entspricht $A_{253}$ = 0,001/min mit BAEE als Substrat bei pH 7,6 und 25 °C bei einem Reaktionsvolumen von 3,2 ml und einer optischen Schichtdicke von 1 cm).

Die eigentliche Bewertung der Aktivität des Trypsinogens (Trypsins) wurde unter Anwendung synthetischer homogener Substrate durchgeführt, die peptidische Analoga natürlicher Substrate darstellen (p-Nitroanilide). Das durch Hydrolyse abgespaltene p-Nitroanilin stellt ein Maß für die Aktivität dar; seine Menge wird spektrophotometrisch bei 405 nm als Extinktionsänderung/min ermittelt und nach der nachstehenden Beziehung in nkat/ml angegeben:

$$\text{nkat} = \frac{A_{405} \times V \times \text{Verdünnung}}{\varepsilon \times v} \times \frac{1000}{60},$$

worin bedeuten:

V das Volumen des Gemischs bei der Messung,
v das Volumen der Probe und
ε den molaren Extinktionskoeffizienten für p-Nitroanilin (10,4).

Die Temperatur bei der Bestimmung betrug 25 °C. Der pH-Wert betrug 7,6 und wurde mit Tris-Ca-Puffer eingestellt. Es wurde das Verhältnis der Aktivitäten nach beiden Bestimmungen ermittelt und graphisch dargestellt. Der Protein-gehalt wurde spektrophotometrisch ermittelt ($A_{280}$). Die spezifische Aktivität (Reinheitsgrad) wurde als Verhältnis von Aktivität zu Proteingehalt angegeben. Dieser Wert charakterisiert das Präparat im Hinblick auf die relative Reinheit des Enzyms.

Das Trypsin wurde mit Hilfe von Z-Gly-Pro-Arg-p-Nitroanilid bestimmt. Chymotrypsin wurde durch Glt-Ala-Ala-Pro-Phe-p-Nitroanilid (-p-NA) bestimmt. Elastase wurde mit Hilfe von Glt-Ala-Ala-Ala-p-NA bestimmt, Kallikrein mit NO-Pro-Phe-Arg-p-NA. Aprotinin wurde als Trypsininhibitor mit Hilfe von Z-Gly-Pro-Arg-p-NA bestimmt.

Da das Trypsinogen die Grundkomponente der Präparate der Beispiele darstellt und das aktive Trypsin alle übrigen im Präparat vorliegenden pankreatischen Proenzyme aktiviert, wurde deren Bestimmung nach der Aktivierung des Trypsinogens mit Hilfe einer Enteropeptidase durchgeführt (vgl. A. Borgström et al., Scand. J. Gastroenterol. 28 (5) (1993) 455).

Definition der Aktivität der Amylasen und Methode zu ihrer Bestimmung:

Als Amylase wude eine kommerzielle α-Amylase (Sigma Co, USA) verwendet. Die Bestimmung der α-Amylase erfolgte aufgrund der Freisetzung von Maltose aus Stärke (1 Einheit setzt 1 mg Maltose aus Stärke während 3 min bei pH 6,9 und 20 °C frei).

Die Amylase (α-Amylase) wurde mit Hilfe eines kommerziellen Tablettentests bestimmt (Hersteller Slovakofarma Hlohovec, SR), der zur Bestimmung von α-Amylasen im Blutserum und in biologischen Flüssigkeiten in der klinischen Analytik verwendet wird. Eine Tablette enthält vernetzte Stärke mit kovalent gebundenem Farbstoff, Aktivator und Puffer. Durch Hydrolyse der unlöslichen farbigen Stärke geht diese in lösliche Form über und wird spektrophotometrisch bei 620 nm bestimmt und in nkat/ml und ggf. in nkat/mg ausgedrückt. Die Beziehung zwischen Absorption bzw. Extinktion und Aktivität wird aus dem entsprechenden Diagramm ermittelt. Das Verfahren wird nach der Anleitung des Herstellers durchgeführt.

Das Verhältnis der nach diesen beiden Methoden bestimmten Aktivität der Amylase wurde experimentell ermittelt und graphisch dargestellt. Die hochgereinigte Amylase wurde als Standard eingesetzt.

Induktion von Geschwülsten durch Methylcholanthren:

Geschwülste wurde bei Mäusen durch Methylcholanthren, das in Olivenöl gelöst verabreicht wurde, künstlich hervorgerufen. Hierzu wurden 100 ml Methylcholanthren unter ständigem Rühren in 50 ml Olivenöl gelöst. Diese Lösung wurde subkutan in einer Dosis von 0,2 ml Öllösung pro Maus appliziert, was 400 µg Methylcholanthren pro Maus entspricht. Die Applikation erfolgte an 3 aufeinanderfolgenden Tagen jeweils an der gleichen Stelle an der rechten Hüfte. Jede Maus erhielt entsprechend eine Gesamtdosis von 1,2 mg Methylcholanthren. Nach der subkutanen Verabreichung dieses karzinogenen Wirkstoffs erscheinen 5-6 Wochen nach der Verabreichung die ersten Geschwülste.

Erzeugung von Melanomen durch Transplantation von Zellen des Melanoms B16:

- Vermehrung der Geschwulstzellen

Die transplantierten Geschwulstzellen wurden in Form des Aszites in der Bauchhöhle vermehrt. Am zehnten Tag nach der intraperionealen Transplantation von $2 \times 10^6$ Geschwulstzellen wurde der Aszites in Henkscher Lösung abgenommen. Die Zellen wurden gezählt. Die Zellsuspension wurde auf eine Konzentration von $2 \times 10^6$ Asziteszellen pro 0,2 ml Suspension verdünnt.

- Transplantation und Entwicklung der Geschwülste und der Metastasen

Die Suspension von Asziteszellen mit einer Konzentration von $2 \times 10^6$ Zellen/0,2 ml wurde intradermal in die linke Hüfte der Mäuse transplantiert. Am zehnten Tag nach der Transplantation des Melanoms B16 wurden die herangewachsenen Geschwülste nach Narkotisierung der Mäuse mit Pentobarbital herausoperiert. Der zehnte Tag für die Exzision der Geschwülste wurde aufgrund von Vorversuchen ausgewählt, in denen die metastatische Aktivität des Malanoms B16 getestet wurde (bei Entnahme der Geschwülste am zehnten Tag nach der Transplantation der Asziteszellen gehen sämtliche Mäuse im Verlauf von 5 Wochen aufgrund der Metastasenwirkung ein).

**Beispiel 1**

Beeinflussung der Primärgeschwulst und der Überlebensdauer:

Im Rahmen dieser Versuche wurde eine Gruppe von Mäusen verwendet, bei denen die Geschwulst durch subkutane Applikation von Methylcholanthren in einer Dosis von 0,2 ml der Öllösung pro Maus, d.h., eine Dosis von 400 µg Methylcholanthren, induziert worden war. Die Applikation erfolgte an drei aufeinanderfolgenden Tagen in die gleiche Stelle an der rechten Hüfte, so daß jede Maus eine Gesamtdosis von 1,2 mg Methylcholanthren erhielt. Die Geschwülste erschienen zwischen dem 37. und dem 44. Tag nach der Applikation des Methylcholanthrens. Am 45. Tag nach der ersten Applikation des Methylcholanthrens wurden die Mäuse in drei Gruppen zu je zehn Mäusen, die eine Behandlung erhielten, und eine Kontrollgruppe von acht Mäusen aufgeteilt. Die Mäuse der Kontrollgruppe erhielten anstelle der verabreichten Zusammensetzungen in gleichen Zeitabständen (24 Stunden) eine physiologische Kochsalzlösung in gleicher Menge (0,1 ml). Die getesteten Wirkstoffe sind mit A und B bezeichnet; sie lagen in eingefrorenem Zustand vor. Die beiden Stoffe wurden unmittelbar vor der Verabreichung geschmolzen und im Verhältnis 1:1 gemischt. Der getestete Wirkstoff wurde den Versuchstieren subkutan an der vom induzierten Tumor entferntesten Stelle in Zeitabständen von 24 h in einer Dosis von 0,1 ml pro Maus subkutan appliziert. Der Kontrollgruppe wurde eine physiologische Kochsalzlösung in gleicher Menge und in gleichen Zeitabständen wie bei den behandelten Versuchsgruppen appliziert.

In Beispiel 1 wurden insgesamt 38 Mäuse verwendet, die in 4 Gruppen augeteilt waren:

Beispiel 1.1.: 10 Mäuse - Applikation eines Gemischs der Lösungen A + B in Grundverdünnung. Substanz A war die Amylase in einer Konzentration von 133,3 Einheiten (Sigma)/ ml, Wirkstoff B war Trypsinogen in einer Konzentration von 15000 BAEE-Einheiten/ml (792 nkat/Z-Gly-Pro-Arg-p-Nitroanilid).

Beispiel 1.2.: 10 Mäuse - Applikation eines Gemischs der Lösungen A + B in einer Verdünnung, die 10x konzentrierter war als die Grundverdünnung.

Beispiel 1.3.: 10 Mäuse - Applikation eines Gemischs der Lösungen A + B in einer Verdünnung, die 30x konzentrierter war als die Grundverdünnung.

Beispiel 1.4.: 8 Mäuse - Kontrollgruppe; Verabreichung einer physiologischen Kochsalzlösung in gleicher Weise und in gleicher Menge.

Die Größe der Geschwülste wurde zweimal wöchentlich gemessen. Das Überleben der Mäuse wurde täglich während einer Dauer von 100 Tagen beobachtet.

Die erhaltenen Ergebnisse sind in den nachstehenden Tabellen und Diagrammen aufgeführt.

## Tabelle 1

Wachstum von durch Methylcholanthren induzierten Geschwülsten bei Applikation der Wirkstoffe A + B gegenüber der Kontrollgruppe

| Tag des Versuchs | Wachstum der mit Methylcholanthren induzierten Geschwülste | | | | | | | |
|---|---|---|---|---|---|---|---|---|
| | Konzentration der applizierten Stoffe A+B | | | | | | Kontrolle | |
| | Beisp. 1.1 | | Beisp. 1.2 | | Beisp. 1.3 | | Beisp. 1.4 | |
| | N | V | N | V | N | V | N | V |
| 1 | 9 (10) | 6,3 | 10 (10) | 10,8 | 7 (10) | 14,0 | 8 (8) | 10,5 |
| 5 | 10 (10) | 44,2 | 10 (10) | 39,1 | 10 (10) | 39,1 | 8 (8) | 34,5 |
| 8 | 10 (10) | 39,1 | 10 (10) | 33,9 | 10 (10) | 34,4 | 8 (8) | 62,9 |
| 12 | 10 (10) | 50,7 | 10 (10) | 47,6 | 9 (10) | 40,3 | 8 (8) | 57,4 |
| 15 | 10 (10) | 56,0 | 10 (10) | 43,8 | 8 (10) | 51,3 | 8 (8) | 80,3 |
| 17 | 10 (10) | 31,9 | 10 (10) | 23,9 | 8 (10) | 38,8 | 8 (8) | 87,1 |
| 19 | 10 (10) | 35,5 | 10 (10) | 40,5 | 8 (10) | 74,0 | 8 (8) | 80,8 |
| 22 | 10 (10) | 48,0 | 10 (10) | 66,2 | 8 (10) | 66,9 | 8 (8) | 98,6 |
| 26 | 10 (10) | 36,7 | 10 (10) | 57,9 | 8 (10) | 94,6 | 8 (8) | 88,5 |
| 29 | 10 (10) | 62,1 | 10 (10) | 50,3 | 8 (10) | 97,6 | 8 (8) | 106,0 |
| 33 | 10 (10) | 74,3 | 10 (10) | 63,5 | 8 (10) | 83,6 | 8 (8) | 114,6 |

| Tag des Versuchs | Wachstum der mit Methylcholanthren induzierten Geschwülste | | | | | | | |
|---|---|---|---|---|---|---|---|---|
| | Konzentration der applizierten Stoffe A+B | | | | | | Kontrolle | |
| | Beisp. 1.1 | | Beisp. 1.2 | | Beisp. 1.3 | | Beisp. 1.4 | |
| | N | V | N | V | N | V | N | V |
| 36 | 10 (10) | 113,0 | 10 (10) | 105,1 | 8 (10) | 150,1 | 9 (9) | 184,0 |
| 40 | 10 (10) | 130,0 | 10 (10) | 148,6 | 9 (10) | 155,9 | 7 (7) | 174,0 |
| 43 | 10 (10) | 120,8 | 10 (10) | 108,2 | 7 (9) | 102,4 | 7 (7) | 201,1 |
| 47 | 10 (10) | 160,7 | 10 (10) | 168,8 | 6 (9) | 126,5 | 7 (7) | 253,1 |
| 50 | 10 (10) | 204,8 | 10 (10) | 196,3 | 6 (9) | 153,2 | 5 (5) | 355,0 |
| 54 | 8 (9) | 205,1 | 10 (10) | 277,5 | 6 (9) | 183,7 | 4 (5) | 388,3 |
| 57 | 8 (9) | 254,8 | 9 (9) | 276,7 | 6 (9) | 213,5 | 3 (4) | 251,3 |
| 61 | 7 (8) | 289,9 | 8 (8) | 328,0 | 6 (9) | 273,5 | 2 (3) | 329,0 |
| 64 | 7 (8) | 301,9 | 6 (7) | 424,3 | 7 (9) | 391,7 | 2 (3) | 332,0 |
| 68 | 6 (6) | 429,8 | 4 (4) | 471,0 | 6 (9) | 443,2 | 0 (3) | – |
| 71 | 4 (4) | 234,0 | 3 (3) | 337,7 | 5 (8) | 429,3 | 0 (0) | – |
| 75 | 4 (4) | 252,5 | 3 (3) | 384,7 | 4 (6) | 439,8 | 0 (0) | – |

Tab. 1: Fortsetzung

| Tag des Versuchs | Wachstum der mit Methylcholanthren induzierten Geschwülste | | | | | | | |
|---|---|---|---|---|---|---|---|---|
| | Konzentration der applizierten Stoffe A + B | | | | | | Kontrolle | |
| | Beisp. 1.1 | | Beisp. 1.2 | | Beisp. 1.3 | | Beisp. 1.4 | |
| | N | V | N | V | N | V | N | V |
| 78 | 4 (4) | 400,5 | 3 (3) | 461,3 | 2 (4) | 656,5 | 0 (0) | − |
| 82 | 3 (3) | 565,0 | 3 (3) | 494,3 | 3 (4) | 836,0 | 0 (0) | − |
| 85 | 3 (3) | 871,7 | 2 (2) | 963,0 | 2 (3) | 565,0 | 0 (0) | − |
| 89 | 3 (3) | 1232 | 1 (1) | 1575 | 2 (3) | 600,5 | 0 (0) | − |
| 92 | 2 (3) | 1567 | 0 (1) | − | 2 (3) | 727,5 | 0 (0) | − |
| 96 | 0 (0) | − | 0 (0) | − | 2 (3) | 790,0 | 0 (0) | − |
| 99 | 0 (0) | − | 0 (0) | − | 1 (2) | 110,0 | 0 (0) | − |
| gestorbene Tiere gesamt | 10 | | 10 | | 8 | | 8 | |
| überlebende Tiere gesamt | 0 | | 0 | | 2 | | 0 | |

N: x (y)

x   − Anzahl der messbaren Geschwülste

(y) − Anzahl der Mäuse in der betreffenden Gruppe

V   − mittlere Größe der Geschwulst

## Tabelle 2

Mortalität der Mäuse mit durch Methylcholanthren induzieren Geschwülsten

| Tag des Versuchs | Mortalität der Mäuse mit durch Methylcholanthren induzierten Geschwülsten | | | | | | Kontrolle Beisp. 1.4 | |
|---|---|---|---|---|---|---|---|---|
| | Konzentration der applizierten Stoffe A+B | | | | | | | |
| | Beisp. 1.1 | | Beisp. 1.2 | | Beisp. 1.3 | | | |
| | N | % zusammen | N | % zusammen | N | % zusammen | N | % zusammen |
| 40 | – | – | – | – | – | – | 1 | 12,5 |
| 43 | – | – | – | – | 1 | 10,0 | – | – |
| 54 | 1 | 10,0 | – | – | – | – | 2 | 37,5 |
| 57 | – | – | 1 | 10,0 | – | – | 1 | 50,0 |
| 61 | 1 | 20,0 | 1 | 20,0 | – | – | 1 | 62,5 |
| 64 | – | – | 1 | 30,0 | – | – | – | – |
| 65 | – | – | – | – | 1 | 20,0 | 1 | 75,0 |
| 66 | 1 | 30,0 | 3 | 60,0 | – | – | 1 | 87,5 |
| 67 | 1 | 40,0 | – | – | – | – | 1 | 100.0 |
| 71 | 2 | 60,0 | 1 | 70,0 | – | – | | |
| 75 | – | – | – | – | 2 | 40,0 | | |

Tab. 2: Fortsetzung

| Tag des Versuchs | Mortalität der Mäuse mit durch Methylcholanthren induzierten Geschwülsten | | | | | | | |
|---|---|---|---|---|---|---|---|---|
| | Konzentration der applizierten Stoffe A+B | | | | | | Kontrolle | |
| | Beisp. 1.1 | | Beisp. 1.2 | | Beisp. 1.3 | | Beisp. 1.4 | |
| | N | % zusammen | N | % zusammen | N | % zusammen | N | % zusammen |
| 78 | – | – | – | – | 2 | 60,0 | | |
| 82 | 1 | 70,0 | – | – | – | – | | |
| 85 | – | – | 1 | 80,0 | 1 | 70,0 | | |
| 89 | – | – | 1 | 90,0 | – | – | | |
| 94 | 1 | 80,0 | – | – | – | – | | |
| 95 | 1 | 90,0 | – | – | – | – | | |
| 96 | 1 | 100,0 | 1 | 100,0 | – | – | | |
| 97 | | | | | 1 | 80,0 | | |
| gestorben | 10 | 100,0 | 10 | 100,0 | 8 | 80,0 | 8 | 100,0 |
| über-lebend | 0 | 0,0 | 0 | 0,0 | 2 | 20,0 | 0 | 0,0 |

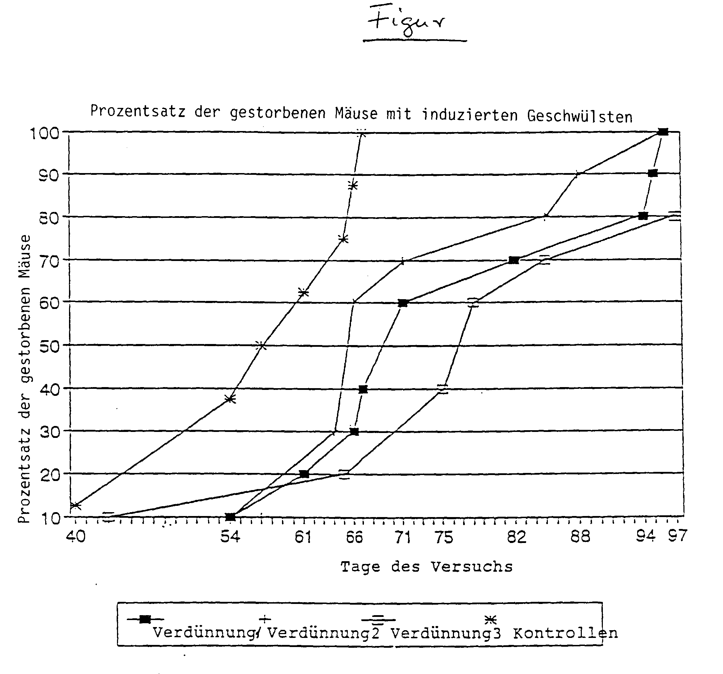

In der Figur sind die Daten der Tabelle 2 graphisch dargestellt.

Die Daten der Tabelle 2 sowie der graphischen Darstellung der Figur zeigen die Wirksamkeit der erfindungsgemäßen pharmazeutischen Mittel gegenüber den Kontrollversuchen, insbesondere hinsichtlich der Verlängerung der Überlebensdauer.

Das Methylcholanthren wurden 50 Mäusen subkutan appliziert. Eine Geschwulst wurde hierdurch bei 38 Mäusen, d.h. bei 76 % der Tiere, induziert. Die Applikation wurde bei den Mäusen vorgenommen, bei denen zwischen dem 37. und 44. Tag nach Verabreichung der ersten Dosis Methylcholanthren Geschwülste aufgetreten waren. Die Mäuse mit Geschwülsten wurden durch zufällige Auswahl in drei Testgruppen und eine Kontrollgruppe aufgeteilt. Die mittlere Größe der Geschwülste am Anfang des Tests betrug 10,4 mm$^2$.

**Beispiel 1.1**

- Wirkung der Wirkstoffe A+B in der Konzentration "1"

Die Versuchsgruppe umfaßte 10 Mäuse. Im Vergleich zur Kontrollgruppe trat das Wachstum der Geschwülste verspätet auf (Tabelle 1). Während bei der Kontrollgruppe die mittlere Größe der Geschwülste den Wert "100" am 29. Tag des Versuchs überschritt, wurde diese Größe bei der behandelten Gruppe erst am 36. Tag, d.h. 7 Tage später, gemessen. Der mittlere Wert der Größe der Geschwulst von "200" wurde bei der Kontrollgruppe am 43. Tag und bei der behandelten Gruppe am 50. Tag festgestellt, also wiederum 7 Tage später.

Noch signifikanter als hinsichtlich der Geschwulstgröße ist die Überlebensdauer der Mäuse in den behandelten Gruppen und der Kontrollgruppe, wie aus Tabelle 2 hervorgeht. Am 27. Tag des Versuchs, an dem 50 % der Mäuse der Kontrollgruppe gestorben waren, überlebten noch 90 % der Mäuse in der behandelten Gruppe. Sämtliche Mäuse der Kontrollgruppe waren bis zum 67. Tag des Versuchs gestorben, während von den Mäusen der behandelten Gruppe an diesem Tag noch 60 % am Leben waren. Das letzte Tier, bei dem das erfindungsgemäße Präparat angewandet worden war, starb am 96. Tag, d.h. 29 Tage später.

**Beispiel 1.2**

- Wirkung der Wirkstoffe A+B in der Konzentration "2"

Die Versuchsgruppe umfaßte 10 Mäuse. Im Vergleich zur Kontrollgruppe trat das Wachstum der Geschwülste verspätet auf (Tabelle 1). Während bei der Kontrollgruppe die mittlere Größe der Geschwülste den Wert "100" am 29. Tag des Versuchs überschritt, wurde diese Größe bei der behandelten Gruppe erst am 36. Tag, d.h. 7 Tage später, gemessen. Der mittlere Wert der Größe der Geschwulst von "200" wurde bei der Kontrollgruppe am 43. Tag und bei der behandelten Gruppe am 50. Tag festgestellt, also wiederum 7 Tage später.

Die Verabreichung der Wirkstoffe A+B in der Konzentration "2" beeinflußte das Wachstum der Geschwülste in gleicher Weise positiv wie in der Konzentration "1".

Noch signifikanter als hinsichtlich der Geschwulstgröße ist die Überlebensdauer der Mäuse in den behandelten Gruppen und der Kontrollgruppe, wie aus Tabelle 2 hervorgeht. Am 27. Tag des Versuchs, an dem 50 % der Mäuse der Kontrollgruppe gestorben waren, überlebten noch 90 % der Mäuse in der behandelten Gruppe. Sämtliche Mäuse der Kontrollgruppe waren bis zum 67. Tag des Versuchs gestorben, während von den Mäusen der behandelten Gruppe an diesem Tag noch 70 % am Leben waren. Das letzte Tier, bei dem das erfindungsgemäße Präparat angewandt worden war, starb am 96. Tag, d.h., 29 Tage später.

**Beispiel 1.3**

- Wirkung der Wirkstoffe A+B in der Konzentration "3"

Die Versuchsgruppe umfaßte 10 Mäuse. Im Vergleich zur Kontrollgruppe trat das Wachstum der Geschwülste verspätet auf (Tabelle 1). Während bei der Kontrollgruppe die mittlere Größe der Geschwülste den Wert "100" am 29. Tag des Versuchs überschritt, wurde diese Größe bei der behandelten Gruppe erst am 36. Tag, d.h., 7 Tage später, gemessen. Der mittlere Wert der Größe der Geschwulst von "200" wurde bei der Kontrollgruppe am 43. Tag und bei der behandelten Gruppe am 57. Tag festgestellt, also wiederum 14 Tage später.

Noch signifikanter als hinsichtlich der Geschwulstgröße ist die Überlebensdauer der Mäuse in den behandelten Gruppen und der Kontrollgruppe, wie aus Tabelle 2 hervorgeht. Am 27. Tag des Versuchs, an dem 50 % der Mäuse der Kontrollgruppe gestorben waren, überlebten noch 90 % der Mäuse in der behandelten Gruppe. Sämtliche Mäuse der Kontrollgruppe waren bis zum 67. Tag des Versuchs gestorben, während von den Mäusen der behandelten Gruppe an diesem Tag noch 80 % am Leben waren. Das letzte Tier, bei dem das erfindungsgemäße Präparat angewandet worden war, starb am 96. Tag, d.h., 29 Tage später. 100 Tage nach Versuchsbeginn überlebten noch 2 Mäuse, d.h., 20 % der Gesamtzahl der behandelten Mäuse.

Die Wirkstoffe A+B waren in der Verdünnung "3" am wirkungsvollsten. Zwei Mäuse wurden hierdurch vollständig geheilt. Bei einer Maus kam es nach 51 Tagen zu einem Relaps; die zweite Maus war am 100. Tag, an dem der Versuch beendet wurde, noch gesund.

**Zusammenfassung**

Aus den oben erläuterten Ergebnissen folgt, daß die Verabreichung der getesteten Wirkstoffe A+B die Überlebensdauer der Mäuse (Inzuchtmäuse C5>B1$_6$) mit chemisch induzierten Geschwülsten in signifikanter Weise positiv beeinflußt. Während die Mäuse der Kontrollgruppe am 67. Tag sämtlich gestorben waren, überlebten von der Versuchsgruppe Nr. 3, welche die höchste Konzentration der verabreichten Stoffe erhielt, noch 20 % der Tiere länger als 100 Tage. Der Einfluß der Wirkstoffe ist aus dem Vergleich der Ergebnisse der einzelnen Gruppen ersichtlich. Der beste Erfolg wurde in der Gruppe mit der höchsten verabreichten Menge erzielt (Beispiel 1.3).

**Beispiel 2**

Beeinflussung der Entstehung und der Entwicklung von Metastasen sowie der Überlebensdauer:

Die Wirkstoffe wurden Mäusen der Zelllinie C5>B1$_6$ appliziert. Das Melanom B16 ist für diese Mäuselinie syngen, was bedeutet, daß bei den Transplantationen keine immunologische Barriere besteht und das Melanom daher eine hohe metastatische Aktivität besitzt.

Die transplantierten Geschwulstzellen wurden in Form des Aszites in der Bauchhöhle vermehrt. Am 10. Tag nach der intraperitonealen Transplatation von 2 x 10$^6$ Geschwulstzellen wurde der Aszites in Henkscher Lösung abgenommen. Die Zellen wurden gezählt; die Konzentration der Suspension wurde auf 2 x 10$^6$ Aszitezellen/0,2 ml Suspension eingestellt. Die Suspension der Aszitezellen wurde intradermal in die linke Hüfte der Mäuse transplantiert. Am 10. Tag nach der Transplantation des Melanoms B16 wurden die angewachsenen Geschwülste nach Narkotisieren der Mäuse mit Pentobarbital herausoperiert. Der zehnte Tag für die Exzision der Geschwülste wurde aufgrund von Vorversuchen ausgewählt, in denen die metastatische Aktivität des Malanoms B16 getestet wurde (bei Entnahme der Geschwülste am zehnten Tag nach der Transplantation der Aszitezellen gehen sämtliche Mäuse im Verlauf von 5 Wochen aufgrund der Metastasenwirkung ein)

Die getesteten Wirkstoffe wurden in eingefrorenem Zustand bei -20 °C gelagert. Unmittelbar vor der Verabreichung wurden sie aufgetaut und temperiert. Die Stoffe A und B (Spezifikation wie in Beispiel 1.1) wurden im Verhältnis 1:1 gemischt. Wirkstoff C (Spezifikation vergl. unten) wurde als solcher verabreicht. Die getesteten Wirkstoffe wurden den Versuchstieren subkutan an einer vom Primärtumor am weitesten entfernten Stelle in einer Dosis von 0,1 oder 0,05 ml pro Maus in Zeitabständen von 24 oder 48 h appliziert. Den Mäusen der Kontrollgruppe wurden die Wirkstoffe A bzw. B allein oder lediglich eine physiologische Kochsalzlösung in gleicher Menge und in gleichen Zeitabschnitten wie bei den Mäusen der behandelten Gruppen appliziert.

Das Überleben der Mäuse wurde täglich über eine Versuchsdauer von 100 Tagen verfolgt. Die gestorbenen Mäuse wurden markiert und bis zur Sektion zur Feststellung der Ursache des Exitus und zum Nachweis von Metastasen in Formalinlösung aufbewahrt.

Die Bezeichnung der Stoffe A und B ist analog zu Beispiel 1. Der Wirkstoff C ist ein teilweise gereinigter Pankreasextrakt. Seine amylolytische Aktivität und sein Trypsinogengehalt sind gleich wie beim Gemisch "A + B". Der Pankreasextrakt enthält ferner noch eine gewisse Menge Chymotrypsinogen.

**Beispiel 2.1.**

Die behandelte Gruppe umfaßte 10 Mäuse. Die getestete Wirkstoffkombination A + B wurde in Zeitabständen von 24 h in einer Menge von 0,1 ml subkutan appliziert. Am 22. Versuchstag waren zwei Mäuse gestorben (20 %) der Gesamtzahl; am 25. Tag starb 1 Maus (10 % der Gesamtzahl); am 28. Tag starb 1 Maus (10 % der Gesamtzahl); am 36. Tag starb 1 Maus (10 % der Gesamtzahl), und am 42. Tag starb 1 Maus (10 % der Gesamtzahl).

4 Mäuse überlebten nach 100 Tagen, d.h., 40 % der Gesamtzahl von 10 behandelten Mäusen.

**Beispiel 2.2**

Die behandelte Gruppe umfaßte 10 Mäuse. Die getestete Wirkstoffkombination A + B wurde in Zeitabständen von 48 h in einer Menge von 0,1 ml subkutan appliziert.

Am 22. Versuchstag starb 1 Maus (10 % der Gesamtzahl); am 34. Tag starb 1 Maus (10 % der Gesamtzahl); am 38. Tag starb 1 Maus (10 % der Gesamtzahl), und am 40. Tag starb 1 Maus (10 % der Gesamtzahl).

6 Mäuse überlebten nach 100 Tagen, d.h., 60 % der Gesamtzahl von 10 behandelten Mäusen.

**Beispiel 2.3**

Die behandelte Gruppe umfaßte 10 Mäuse. Die getestete Wirkstoffkombination A + B wurde in Zeitabständen von 48 h in einer Menge von 0,015 ml subkutan appliziert.

Am 12. Versuchstag starb 1 Maus (10 % der Gesamtzahl); am 16. Tag starb 1 Maus (10 % der Gesamtzahl); am 23. Tag starben 2 Mäuse (20 % der Gesamtzahl); am 28. Tag starb 1 Maus (10 % der Gesamtzahl); am 33. Tag starb 1 Maus (10 % der Gesamtzahl); am 35. Tag starb 1 Maus (10 % der Gesamtzahl), und am 39. Tag starb ebenfalls 1 Maus (10 % der Gesamtzahl).

2 Mäuse überlebten nach 100 Tagen, d.h., 20 % der Gesamtzahl von 10 behandelten Mäusen.

**Beispiel 2.4**

- Grundkontrollgruppe

Die Kontrollgruppe bestand aus 6 Mäusen. Diesen Tieren wurde lediglich physiologische Kochsalzlösung in Zeitabständen von 48 Stunden in einer Menge von 0,1 ml appliziert. Am 17. Versuchstag starb 1 Maus (16,6 % der Gesamtzahl); am 19. Tag starben 2 Mäuse (33,2 % der Gesamtzahl); am 23., 29. und 34. Tag des Versuchs starb jeweils 1 Maus (d.h. jedes mal 16,6 % der Gesamtzahl).

Nach 100 Tagen überlebte keine Maus, d.h., zu diesem Zeitpunkt waren sämtliche Tiere der Kontrollgruppe, also 100 % der Gesamtzahl, gestorben.

**Beispiel 2.5**

- Erste Kontrollgruppe

Die Kontrollgruppe umfaßte 10 Mäuse. Diesen Tieren wurde lediglich der Wirkstoff A allein in Zeitabständen von 48 h in einer Menge von 0,05 ml subkutan appliziert. Am 24. Versuchstag war 1 Maus gestorben (10 % der Gesamtzahl); am 26. Tag starb 1 Maus (10 % der Gesamtzahl); am 27. Tag starb 1 Maus (10 % der Gesamtzahl); am 33. Tag starb 1 Maus (10 % der Gesamtzahl); am 35. Tag starb 1 Maus (10 % der Gesamtzahl); am 37. Tag starb 1 Maus (10 % der Gesamtzahl); am 39. Tag starb 1 Maus (10 % der Gesamtzahl), und am 47. Tag des Versuchs starb 1 Maus (10 % der Gesamtzahl).

2 Mäuse überlebten nach 100 Tagen, d.h., 20 % der Gesamtzahl von 10 Tieren dieser Kontrollgruppe.

**Beispiel 2.6**

- Zweite Kontrollgruppe

Die Kontrollgruppe bestand aus 10 Mäusen. Die Versuchstiere erhielten lediglich den Wirktstoff B allein in Zeitabständen von 48 h in einer Menge von 0,05 ml subkutan appliziert. Am 23. Versuchstag waren 2 Mäuse gestorben (20 % der Gesamtzahl); am 25. Tag des Versuchs starb 1 Maus (10 % der Gesamtzahl); am 26. Tag des Versuchs starb 1 Maus (10 % der Gesamtzahl); am 30. Tag des Versuchs starb 1 Maus (10 % der Gesamtzahl); am 31. Tag starb 1 Maus (10 % der Gesamtzahl); am 34. Tag des Versuchs starb 1 Maus (10 % der Gesamtzahl), und am 50. Tag des Versuchs starb 1 Maus (10 % der Gesamtzahl).

2 Mäuse überlebten nach 100 Tagen, d.h., 20 % der Gesamtzahl der Tiere dieser Kontrollgruppe.

**Beispiel 2.7**

Die behandelte Gruppe umfaßte 10 Mäuse. Der Wirkstoff C wurde in Zeitabständen von 48 h in einer Menge von 0,1 ml subkutan appliziert. Am 36. Tag des Versuchs starb 1 Maus (10 % der Gesamtzahl); am 38. Tag des Versuchs starb 1 Maus (10 % der Gesamtzahl); am 42. Tag des Versuchs starb 1 Maus (10 % der Gesamtzahl), und am 43. Tag starb 1 Maus (10 % der Gesamtzahl).

6 Mäuse überlebten nach 100 Tagen, d.h., 60 % der Gesamtzahl von 10 behandelten Mäusen.

**Beispiel 2.8**

- Dritte Kontrollgruppe

Die Kontrollgruppe umfaßte 14 Mäuse. Die Tiere erhielten lediglich physiologische Kochsalzlösung in Zeitabständen von 48 h in einer Menge von 0,1 ml subkutan appliziert. Am 5. Tag des Versuchs starb 1 Maus (7,1 % der Gesamtzahl); am 15. Tag starben 2 Mäuse (14,3 % der Gesamtzahl); am 19. Tag des Versuchs starb 1 Maus (7,1 % der Gesamtzahl); am 20., 21., 22., 23., 27. und 29. Tag des Versuchs starb je 1 Maus (jeweils 7,1 % der Gesamtzahl); am 30. Tag des Versuchs starben 2 Mäuse (14,3 % der Gesamtzahl), und am 31. und 32. Tag des Versuchs starb jeweils 1 Maus (d.h., jeweils 7,1 % der Gesamtzahl).

Nach 100 Tagen war keine Maus mehr am Leben, d.h., die Gesamtzahl der Mäuse dieser Versuchsgruppe, also 100 %, waren zu diesem Zeitpunkt gestorben.

**Beispiel 2.9**

- Kontrollgruppe

Die Kontrollgruppe umfaßte 14 Mäuse. In dieser Kontrollgruppe wurde lediglich das Überleben der Mäuse nach der Exzision des Melanoms B16 verfolgt. Die Geschwülste wurden am 10. Tag nach der intradermalen Transplantation von $2 \times 10^6$ Asziteszellen herausoperiert.

Die Ergebnisse bezüglich der Überlebensdauer bzw. der Mortalität sind in den nachstehenden Tabellen 3 und 4 zusammengefaßt.

Tabelle 4 bezieht sich auf die Mortalität der Mäuse der Kontrollgruppe nach Exzision der Primärgeschwulst. In diesem Fall überlebte keine Maus 100 Tage, die Überlebensrate betrug also 0 %.

## Tabelle 3

Ergebnisse der Beispiele 2.1 - 2.8

| Beispiel Nr. | Anzahl der Mäuse | applizierter Stoff | | | Anzahl der Exitus | | nach 100 Tagen überlebende Tiere | überlebende Tiere (%) |
|---|---|---|---|---|---|---|---|---|
| | | Typ | Dosis (ml) | Zeitabstand (Stunden) | zu-sammen | Tag-Anzahl | | |
| 2.1 | 10 | A+B | 0,1 | 24 | 6 | 22-2 | 4 | 40 |
| | | | | | | 25-1 | | |
| | | | | | | 29-1 | | |
| | | | | | | 36-1 | | |
| | | | | | | 42-1 | | |
| 2.2 | 10 | A+B | 0,1 | 48 | 4 | 22-1 | 6 | 60 |
| | | | | | | 34-1 | | |
| | | | | | | 38-1 | | |
| | | | | | | 40-1 | | |
| 2.3 | 10 | A+B | 0,05 | 48 | 8 | 12-1 | 2 | 20 |
| | | | | | | 16-1 | | |
| | | | | | | 23-2 | | |
| | | | | | | 29-1 | | |
| | | | | | | 33-1 | | |
| | | | | | | 35-1 | | |
| | | | | | | 39-1 | | |
| 2.4 | 6 | phys. Lösung | 0,1 | 48 | 6 | 17-1 | 0 | 0 |
| | | | | | | 19-2 | | |

Fortsetzung
Tabelle 3: Ergebnisse der Beispiele 2.1 - 2.8

| Beispiel Nr. | Anzahl der Mäuse | applizierter Stoff | | | Anzahl der Exitus | | nach 100 Tagen überlebende Tiere | überlebende Tiere (%) |
| | | Typ | Dosis (ml) | Zeitabstand (Stunden) | zusammen | Taganzahl | | |
|---|---|---|---|---|---|---|---|---|
| | | | | | | 23-1 | | |
| | | | | | | 29-1 | | |
| | | | | | | 35-1 | | |
| 2.5 | 10 | A | 0,05 | 48 | 8 | 24-1 | 2 | 20 |
| | | | | | | 26-1 | | |
| | | | | | | 27-1 | | |
| | | | | | | 33-1 | | |
| | | | | | | 35-1 | | |
| | | | | | | 37-1 | | |
| | | | | | | 39-1 | | |
| | | | | | | 47-1 | | |
| 2.6 | 10 | B | 0,05 | 48 | 8 | 23-2 | 2 | 20 |
| | | | | | | 25-1 | | |
| | | | | | | 26-1 | | |
| | | | | | | 30-1 | | |
| | | | | | | 31-1 | | |
| | | | | | | 34-1 | | |
| | | | | | | 50-1 | | |
| 2.7 | 10 | C | 0,1 | 48 | 4 | 36-1 | 6 | 60 |
| | | | | | | 38-1 | | |

Tabelle 3: Fortsetzung - Ergebnisse der Beispiele 2.1 - 2.8

| Beispiel Nr. | Anzahl der Mäuse | applizierter Stoff | | | Anzahl der Exitus | | nach 100 Tagen überlebende Tiere | überlebende Tiere (%) |
|---|---|---|---|---|---|---|---|---|
| | | Typ | Dosis (ml) | Zeitab-stand | zu-sammen | Tag-Anzahl | | |
| | | | | | | 42-1 | | |
| | | | | | | 43-1 | | |
| 2.8 | 14 | phys. Lösung | 0,1 | 48 | 14 | 5-1 | 0 | 0 |
| | | | | | | 15-2 | | |
| | | | | | | 19-1 | | |
| | | | | | | 20-1 | | |
| | | | | | | 21-1 | | |
| | | | | | | 22-1 | | |
| | | | | | | 23-1 | | |
| | | | | | | 27-1 | | |
| | | | | | | 29-1 | | |
| | | | | | | 30-2 | | |
| | | | | | | 31-1 | | |
| | | | | | | 32-1 | | |

Tabelle 4

| Mortalität der Tiere der Kontrollgruppe aufgrund von Metastasen nach Exzision der Primärgeschwulst | | | |
|---|---|---|---|
| Tag des Versuchs | Anzahl der Exitus | gestorbene Tiere (%) | überlebende Tiere (%) |
| 5 | 1 | 7,14 | 92,86 |
| 15 | 2 | 21,43 | 78,57 |
| 19 | 1 | 28,57 | 71,43 |
| 20 | 1 | 35,71 | 64,29 |
| 21 | 1 | 42,86 | 57,14 |
| 22 | 1 | 50,00 | 50,00 |
| 23 | 1 | 57,14 | 42,86 |
| 27 | 1 | 64,29 | 35,71 |
| 29 | 1 | 71,43 | 28,57 |
| 30 | 2 | 85,71 | 14,29 |

Tabelle 4   (fortgesetzt)

| Mortalität der Tiere der Kontrollgruppe aufgrund von Metastasen nach Exzision der Primärgeschwulst | | | |
|---|---|---|---|
| Tag des Versuchs | Anzahl der Exitus | gestorbene Tiere (%) | überlebende Tiere (%) |
| 31 | 1 | 92,86 | 7,14 |
| 32 | 1 | 100,00 | 0,00 |
| Anzahl der Exitus der 14 Mäuse | 14 | 100,00 | 0,00 |

## Zusammenfassung

Aus den obigen Ergebnissen folgt, daß die Kombination der Wirkstoffe "A + B" sowie der Wirkstoff "C" zu einer Inhibierung der Entstehung von Metastasen des Melanoms B16 führen. Im Fall der Wirkstoffkombination A + B erwiesen sich die Dosis von 0,2 ml/20 g und der Zeitabstand der Verabreichungen von 48 h besonders günstig, da in diesem Fall 60 % der Versuchstiere länger als 100 Tage überlebten, während die Tiere der ersten Kontrollgruppe des Beispiels 2.5 aufgrund von Metastasen bis zum 35. Versuchstag und die Tiere der Kontrollgruppe des Beispiels 2.9 bis zum 32. Versuchstag gestorben waren.

Aufgrund des Umstands, daß das Melanom B16 bei den verwendeten Inzuchtmäusen C5 > B16 eine besonders aggressive, metastasierende Geschwulst darstellt, zeigen die erhaltenen Ergebnisse bei Verwendung der Wirkstoffkombination aus Amylase + Trypsinogen aus Pankreasextrakt besonders deutlich die vorteilhafte Wirksamkeit der erfindungsgemäßen pharmazeutischen Mittel. Die erfindungsgemäßen pharmazeutischen Mittel, insbesondere diejenigen, in denen die Wirkstoffkombination A + B in gemischter Form bzw. in Form des Stoffes C vorliegt, eignen sich daher besonders zur Krebstherapie und zur Behandlung maligner Geschwülste in der Human- und Veterinärmedizin.

## Patentansprüche

1. Pharmazeutische Mittel, insbesondere mit Modulationseffekt auf maligne Geschwülste,
   dadurch gekennzeichnet, daß sie als Wirkstoff ein oder mehrere Protease-Proenzyme enthalten.

2. Pharmazeutische Mittel nach Anspruch 1, dadurch gekennzeichnet, daß sie ferner eine oder mehrere Amylasen enthalten, insbesondere $\alpha$-Amylasen.

3. Pharmazeutische Mittel nach Anspruch 2, dadurch gekennzeichnet, daß das oder die Protease-Proenzyme und die Amylase(n) in einem solchen Mengenverhältnis vorliegen, daß das Verhältnis der enzymatischen Aktivität der Protease-Proenzyme zur enzymatischen Aktivität der Amylase(n), jeweils ausgedrückt in entsprechenden Einheiten der enzymatischen Aktivität, im Bereich von 1:100 bis 100:1 liegt.

4. Pharmazeutische Mittel nach einem oder mehreren der Ansprüche 1 bis 3, dadurch gekennzeichnet, daß die Protease-Proenzyme ausgewählt sind unter

   Trypsinogen,
   Chymotrypsinogen,
   Proelastase und
   Prokallikrein.

5. Pharmazeutische Mittel nach einem oder mehreren der Ansprüche 1 bis 4, dadurch gekennzeichnet, daß sie eine oder mehrere Proteasen in einer nichtaktiven Form enthalten.

6. Pharmazeutische Mittel nach einem oder mehreren der Ansprüche 1 bis 5, dadurch gekennzeichnet, daß die Protease-Proenzyme menschlichen oder tierischen Ursprungs sind.

7. Pharmazeutische Mittel nach einem oder mehreren der Ansprüche 1 bis 6, dadurch gekennzeichnet, daß die Amylasen menschlichen, tierischen, pflanzlichen oder bakteriellen Ursprungs sind.

8. Pharmazeutische Mittel nach einem oder mehreren der Ansprüche 1 bis 7, dadurch gekennzeichnet, daß sie einen Proteaseinhibitor, vorzugsweise einen polyvalenten Proteaseinhibitor, enthalten.

9. Pharmazeutische Mittel nach Anspruch 8, dadurch gekennzeichnet, daß sie als Proteaseinhibitor Aprotinin enthalten.

10. Pharmazeutische Mittel nach einem oder mehreren der Ansprüche 1 bis 9, dadurch gekennzeichnet, daß sie neben den Protease-Proenzymen und gegebenenfalls Amylase(n) pharmakologisch übliche Hilfs- und/oder Trägerstoffe sowie gegebenenfalls weitere, von Protease-Proenzymen und Amylasen verschiedene Wirkstoffe enthalten.

11. Pharmazeutische Mittel nach einem oder mehreren der Ansprüche 1 bis 10, dadurch gekennzeichnet, daß sie in Form von Infusionslösungen oder von Injektionslösungen, insbesondere zur subkutanen Injektion, formuliert sind.

12. Pharmazeutische Mittel nach einem oder mehreren der Ansprüche 2 bis 11, dadurch gekennzeichnet, daß sie als pharmazeutische Kits-of-Parts konfektioniert sind, die eine erste Packungseinheit, die das oder die Protease-Proenzyme und die Amylase (n) und eine zweite Packungseinheit enthalten, die pharmazeutische Hilfs- und/oder Trägerstoffe, insbesondere ein Lösungsmittel zur Rekonstituierung von Infusions- oder Injektionslösungen, enthält.

13. Verfahren zur Herstellung der pharmazeutischen Mittel nach den Ansprüchen 1 bis 10, dadurch gekennzeichnet, daß der bzw. die Wirkstoffe in an sich bekannter Weise, gegebenenfalls zusammen mit üblichen Hilfs- und/oder Trägerstoffen, zu galenischen Formulierungen verarbeitet werden, insbesondere zu Injektionslösungen.

14. Verwendung von Protease-Proenzymen, insbesondere von Trypsinogen, Chymotrypsinogen, Proelastase und/oder Prokallikrein, gegebenenfalls in Kombination mit einer oder mehreren Amylasen, zur Herstellung pharmazeutischer Mittel zur Krebstherapie, besonders zur Behandlung maligner Geschwülste.

15. Verfahren zur Krebstherapie, insbesondere zur Behandlung maligner Geschwülste oder maligner Zellen bzw. Zellkulturen, unter Ausschluß von zu therapeutischen Zwecken durchgeführten Verfahren zur Behandlung des menschlichen oder tierischen Körpers, gekennzeichnet durch Anwendung eines oder mehrerer Protease-Proenzyme, gegebenenfalls in Kombination mit einer oder mehreren Amylasen, auf die malignen Geschwülste, Zellen bzw. Zellkulturen.

Figur

Prozentsatz der gestorbenen Mäuse mit induzierten Geschwülsten